(19) Europäisches Patentamt
European Patent Office
Office européen des brevets

(11) EP 2 630 151 B1

(12) **FASCICULE DE BREVET EUROPEEN**

(45) Date de publication et mention de la délivrance du brevet:
**12.04.2017 Bulletin 2017/15**

(21) Numéro de dépôt: **11770443.7**

(22) Date de dépôt: **17.10.2011**

(51) Int Cl.:
***C07F 5/02*** (2006.01) ***G01N 21/64*** (2006.01)

(86) Numéro de dépôt international:
**PCT/EP2011/068107**

(87) Numéro de publication internationale:
**WO 2012/052399 (26.04.2012 Gazette 2012/17)**

(54) **UTILISATION D'AZABORONATES CYCLIQUES COMME MATERIAUX SENSIBLES DANS DES CAPTEURS DESTINES A DETECTER LA PRESENCE DE PEROXYDES DANS UN MILIEU GAZEUX**

VERWENDUNG VON CYCLISCHEN AZABORONATEN ALS EMPFINDLICHE MATERIALIEN BEI SENSOREN ZUM NACHWEIS VON PEROXIDEN IN EINER GASFÖRMIGEN UMGEBUNG

USE OF CYCLIC AZABORONATES AS SENSITIVE MATERIALS IN SENSORS INTENDED TO DETECT THE PRESENCE OF PEROXIDES IN A GASEOUS ENVIRONMENT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **19.10.2010 FR 1058530**

(43) Date de publication de la demande:
**28.08.2013 Bulletin 2013/35**

(73) Titulaire: **Commissariat à l'Énergie Atomique et aux Énergies Alternatives**
**75015 Paris (FR)**

(72) Inventeurs:
- **CARON, Thomas**
  **F-37520 La Riche (FR)**
- **BOUHADID, Myriam**
  **F-37300 Joue-les-tours (FR)**
- **PASQUINET, Eric**
  **F-37550 Saint Avertin (FR)**
- **MONTMEAT, Pierre**
  **F-37520 La Riche (FR)**
- **SEREIN-SPIRAU, Françoise**
  **F-34090 Montpellier (FR)**

(74) Mandataire: **Ilgart, Jean-Christophe et al**
**BREVALEX**
**95, rue d'Amsterdam**
**75378 Paris Cedex 8 (FR)**

(56) Documents cités:
**EP-A2- 1 291 347 WO-A1-83/04255**
**WO-A1-03/105860 WO-A1-2004/041833**
**WO-A2-03/059312 GB-A- 877 874**
**US-A- 2 939 877 US-A- 2 970 130**
**US-A- 4 659 817 US-A1- 2008 319 204**

- **BOUILLON A ET AL: "Synthesis of novel halopyridinylboronic acids and esters. Part 4: Halopyridin-2-yl-boronic acids and esters are stable, crystalline partners for classical Suzuki cross-coupling", TETRAHEDRON, ELSEVIER SCIENCE PUBLISHERS, AMSTERDAM, NL, vol. 59, no. 50, 8 décembre 2003 (2003-12-08), pages 10043-10049, XP004474451, ISSN: 0040-4020, DOI: DOI:10.1016/J.TET.2003.10.020**
- **ROY ET AL: "Stability of boronic esters - Structural effects on the relative rates of transesterification of 2-(phenyl)-1,3,2-dioxaborolane", JOURNAL OF ORGANOMETALLIC CHEMISTRY, ELSEVIER-SEQUOIA S.A. LAUSANNE, CH, vol. 692, no. 4, 4 janvier 2007 (2007-01-04), pages 784-790, XP005822775, ISSN: 0022-328X, DOI: DOI:10.1016/J.JORGANCHEM.2006.10.013**
- **ALLEN L M ET AL: "Studies on aryl boronic acids. I. Synthesis of naphthalene-1,4-diboronic acid.", JOURNAL OF PHARMACEUTICAL SCIENCES MAR 1969 LNKD- PUBMED:5782037, vol. 58, no. 3, mars 1969 (1969-03), pages 368-369, XP002632195, ISSN: 0022-3549**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 630 151 B1

- **DALE WESLEY J ET AL: "Substituted styrenes. VII. Syntheses and some reactions of the vinylbenzeneboronic acids", JOURNAL OF ORGANIC CHEMISTRY, AMERICAN CHEMICAL SOCIETY, EASTON.; US, vol. 27, 1 janvier 1962 (1962-01-01), pages 2598-2603, XP002557301, ISSN: 0022-3263**
- **KALININ ALEXEY V ET AL: "Di(isopropylprenyl)borane: A new hydroboration reagent for the synthesis of alkyl and alkenyl boronic acids.", ANGEWANDTE CHEMIE (INTERNATIONAL ED. IN ENGLISH) 28 JUL 2003 LNKD- PUBMED:12888969, vol. 42, no. 29, 28 juillet 2003 (2003-07-28), pages 3399-3404, XP002632196, ISSN: 1433-7851**

## Description

### DOMAINE TECHNIQUE

La présente invention se rapporte à l'utilisation d'azaboronates cycliques comme matériaux sensibles dans des capteurs destinés à détecter la présence de peroxydes, et en particulier du peroxyde d'hydrogène, dans un milieu gazeux.

Elle se rapporte également à de nouveaux azaboronates cycliques ainsi qu'à des capteurs comprenant ces azaboronates comme matériaux sensibles.

Le peroxyde d'hydrogène étant un composé à partir duquel il est possible de préparer artisanalement des explosifs comme le triperoxyde de triacétone (TATP) ou le triperoxyde d'hexaméthylène diamine (HMDT), ainsi qu'un produit de décomposition de ces explosifs, l'invention trouve notamment application dans la lutte contre le terrorisme.

Les peroxydes étant, par ailleurs, des composés très instables qui se décomposent facilement en libérant, pour un certain nombre d'entre eux, des vapeurs inflammables, l'invention trouve également application dans la surveillance, à des fins sécuritaires, de locaux dans lesquels sont fabriqués, stockés et/ou utilisés des peroxydes ou des composés peroxydables, c'est-à-dire des composés qui, initialement, ne sont pas des peroxydes mais qui sont susceptibles de se transformer au moins partiellement en peroxydes suite à un phénomène d'auto-oxydation, encore appelé peroxydation, ainsi que dans la surveillance de la pollution atmosphérique.

### ÉTAT DE LA TECHNIQUE ANTÉRIEURE

Les peroxydes sont des composés chimiques qui comprennent un ou plusieurs groupes -O-O- et qui ont, de ce fait, un fort pouvoir oxydant.

Ils sont donc couramment utilisés comme agents blanchissants, notamment dans l'industrie textile, pour le blanchiment de fibres naturelles comme les fibres de coton, et dans l'industrie papetière, pour le blanchiment de la pâte à papier.

Ils sont également couramment utilisés comme initiateurs, promoteurs ou catalyseurs dans des procédés de polymérisation radicalaire, ainsi que comme agents de réticulation ou de vulcanisation dans l'industrie des matières plastiques.

A cela, il faut ajouter des utilisations propres à chaque peroxyde.

Ainsi, par exemple, le peroxyde d'hydrogène, de formule $H_2O_2$, encore connu sous le nom d'eau oxygénée, est employé comme :

- agent de désinfection ;
- agent de stérilisation, notamment dans l'industrie alimentaire où, vaporisé à haute température, il sert à stériliser les emballages composites juste avant l'incorporation des produits alimentaires, et dans l'industrie médicale où il sert à stériliser des dispositifs médicaux thermosensibles et, donc, non stérilisables par la chaleur ;
- agent de traitement d'eaux résiduaires domestiques ou industrielles ; et comme
- agent de traitement d'effluents gazeux.

Il se trouve que le peroxyde d'hydrogène est aussi susceptible d'être utilisé pour fabriquer de manière artisanale des explosifs comme le TATP ou l'HMTD et que ces explosifs donnent, lorsqu'ils se décomposent, du peroxyde d'hydrogène.

Il se trouve également que les peroxydes ont la particularité d'être des composés très instables et de se décomposer en libérant, pour un certain nombre d'entre eux, des vapeurs inflammables.

Il est donc hautement souhaitable de pouvoir disposer de dispositifs permettant de détecter de manière fiable mais néanmoins rapide la présence de peroxydes, notamment lorsqu'ils sont à l'état de vapeurs, que ce soit pour déjouer une menace terroriste ou pour prévenir tout risque d'accident dans des locaux où sont fabriqués, stockés et/ou utilisés des peroxydes ou des composés peroxydables.

Depuis un certain nombre d'années, le développement de capteurs capables de détecter en temps réel des espèces chimiques est en plein essor. Le fonctionnement de ces capteurs est basé sur l'utilisation d'un matériau sensible, c'est-à-dire d'un matériau dont au moins une propriété physique est modifiée au contact des espèces chimiques recherchées, qui est relié à un système apte à mesurer instantanément toute variation de cette propriété physique et de mettre, ainsi, en évidence la présence des espèces chimiques recherchées.

Les avantages des capteurs chimiques sont multiples : instantanéité des résultats, possibilité de miniaturisation et, donc, portabilité, maniabilité et autonomie importante, faibles coûts de fabrication et d'exploitation, etc.

Toutefois, il est évident que leurs performances sont extrêmement variables selon la nature du matériau sensible utilisé.

WO 83/04255 décrit des composés répondant à deux formules chimiques différentes, dites formule (1) et formule (2), dont certains sont des azaboronates, ainsi que l'utilisation des composés de formule (1) pour doser, de manière indirecte et par fluorescence ou colorimétrie, la présence en solution aqueuse d'un composé qui, comme le glucose, est capable de libérer un peroxyde par réaction enzymatique. Il n'est nullement question dans ce document de détecter la présence de peroxydes dans un milieu gazeux.

Les Inventeurs se sont donc fixé pour but de trouver des composés qui soient capables de réagir à la présence de peroxydes se trouvant à l'état de vapeurs.

Ils se sont également fixé pour but que ces composés réagissent très rapidement à cette présence.

Ils se sont de plus fixé pour but que ces composés soient utilisables sous la forme de films minces de manière à pouvoir être utilisés dans des capteurs de faibles dimensions, aisément transportables et utilisables sur tout type de sites.

Ils se sont en outre fixé pour but que ces composés soient relativement simples à synthétiser et que leur synthèse ne fasse appel qu'à des réactions classiquement utilisées en chimie organique.

Or, dans le cadre de leurs travaux, les Inventeurs ont constaté que des azaboronates cycliques satisfont à toutes ces exigences et sont donc susceptibles de constituer des matériaux sensibles de choix dans des capteurs destinés à détecter la présence de ces peroxydes dans un milieu gazeux.

## EXPOSÉ DE L'INVENTION

L'invention a donc, en premier lieu, pour objet l'utilisation d'un azaboronate répondant à la formule générale (I) ci-après :

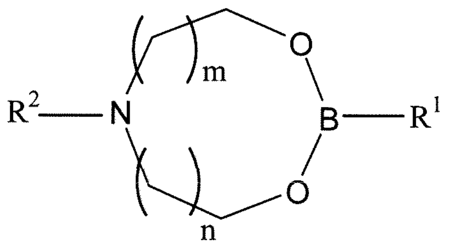

(I)

dans laquelle :

m et n valent, indépendamment l'un de autre, 1 ou 2 ;

$R^1$ représente un groupe alkyle comprenant de 1 à 6 atomes de carbone, un groupe hétérocycloalkyle comprenant de 1 à 3 cycles à 5 ou 6 chaînons chacun, un groupe aryle ou hétéroaryle comprenant de 1 à 3 cycles à 5 ou 6 chaînons chacun, un groupe arylalkyle ou hétéroarylalkyle dont le radical alkyle comprend de 1 à 6 atomes de carbone et dont le radical aryle ou hétéroaryle comprend de 1 à 3 cycles à 5 ou 6 chaînons chacun ; tandis que

$R^2$ représente un atome d'hydrogène ou un groupe alkyle comprenant de 1 à 6 atomes de carbone, un groupe hétérocycloalkyle comprenant de 1 à 3 cycles à 5 ou 6 chaînons chacun, un groupe aryle ou hétéroaryle comprenant de 1 à 3 cycles à 5 ou 6 chaînons chacun, un groupe arylalkyle ou hétéroarylalkyle dont le radical alkyle comprend de 1 à 6 atomes de carbone et dont le radical aryle ou hétéroaryle comprend de 1 à 3 cycles à 5 ou 6 chaînons chacun ;

comme matériau sensible dans un capteur destiné à détecter la présence d'un peroxyde dans un milieu gazeux par mise en contact de ce capteur avec ce milieu gazeux.

Dans la formule générale (I), le doublet libre de l'azote peut, en fonction de la conformation de l'azaboronate, combler la lacune électronique de l'atome de bore, auquel cas ces atomes d'azote et de bore sont liés l'un à l'autre par une liaison dative.

Les groupes représentés par $R^1$ et par $R^2$ peuvent être substitués une ou plusieurs fois.

Ainsi, les groupes représentés par $R^1$ et par $R^2$ peuvent notamment être :

- les groupes alkyle, linéaires ou ramifiés suivants : méthyle, éthyle, *n*-propyle, *i*-propyle, *n*-butyle, *i*-butyle, *s*-butyle, *t*-butyle, *n*-pentyle, *i*-pentyle, *n*-hexyle, etc ; ou
- les groupes hétérocycloalkyle suivante dioxolane, dioxane, dithiolane, thioxolane, thioxane, pipérazinyle, pipéridinyle, pyrrolidinyle, imidazolylidinyle, pyrazolidinyle, tétrahydrofuryle, tétrahydrothiényle, indolinyle, thioazolidinyle, etc ; ou
- les groupes aryle suivants : phényle, naphtyle, acénaphtyle, fluorényle, anthracényle, etc ; ou

- les groupes hétéroaryle suivants : furyle, thiényle, pyrrolyle, oxazolyle, pyrazolyle, thiazolyle, phénothiazolyle, benzothiazolyle, benzofuryle, imidazolyle, benzimidazolyle, triazolyle, pyridyle, pyranyle, quinolinyle, isoquinolinyle, pyrazinyle, pyrimidinyle, carbazolyle, phénothiazolyle, acridinyle, thioxanthényle, pyridyloxazolyle, benzoxazolyle, benzoxadiazolyle, etc ; ou
- les groupes arylalkyle (encore connus sous le nom de groupes aralkyle), c'est-à-dire comprenant un groupe alkyle substitué par un groupe aryle, suivants : benzyle, phénéthyle, anthracénylméthyle, anthracényléthyle, naphtylméthyle, naphtyl-éthyle, etc ; ou
- les groupes hétéroarylalkyle (encore connus sous le nom de groupes hétéroaralkyle), c'est-à-dire comprenant un groupe alkyle substitué par un groupe hétéroaryle, suivants ; carbazolylméthyle, carbazolyléthyle, pyridylméthyle, pyridyléthyle, thiénylméthyle, thiényléthyle, thiazolylméthyle, thiazolyléthyle, etc.

Conformément à l'invention, l'un de $R^1$ et de $R^2$ peut être un groupe fluorescent, c'est-à-dire capable d'émettre un signal lumineux en réponse à une excitation lumineuse appropriée.

Conformément à l'invention, on préfère que $R^1$ représente un groupe phényle, un groupe phényle substitué par un ou plusieurs atomes d'halogène, de préférence, de brome, un groupe benzofuryle, un groupe phénéthyle, un groupe phénéthyle substitué par un groupe phénoxy, un groupe pyridyle, un groupe carbazolyléthyle, un groupe tétrahydrothiényle ou un groupe tétrahydrothiényle dont l'atome de soufre est lié à deux atomes d'oxygène (auquel cas cet atome de soufre forme un groupe sulfoxyde avec ces deux atomes d'oxygène), tandis que l'on préfère que $R^2$ représente un atome d'hydrogène, un groupe alkyle comprenant de 1 à 4 atomes de carbone, un groupe phényle, un groupe naphtylméthyle ou un groupe anthracénylméthyle.

Ainsi, des azaboronates particulièrement préférés sont ceux qui répondent à la formule générale (I) dans laquelle m et n valent 1 et dans laquelle :

- $R^1$ représente un groupe phényle tandis que $R^2$ représente un groupe éthyle, ou
- $R^1$ représente un groupe phényle substitué par un atome de brome, et en particulier un groupe 3-bromophényle ou 4-bromophényle, tandis que $R^2$ représente un groupe n-butyle, ou
- $R^1$ représente un groupe phényle tandis que $R^2$ représente un groupe anthracénylméthyle, ou
- $R^1$ représente un groupe benzofuryle, et en particulier un groupe 2-benzofuryle, tandis que $R^2$ représente un groupe anthracénylméthyle, ou
- $R^1$ représente un groupe phényle tandis que $R^2$ représente un groupe *n*-butyle, ou
- $R^1$ représente un groupe pyridyle tandis que $R^2$ représente un groupe phényle, ou
- $R^1$ représente un groupe phényle tandis que $R^2$ représente un groupe naphtylméthyle, ou
- $R^1$ représente un groupe phénéthyle tandis que $R^2$ représente un groupe éthyle, ou
- $R^1$ représente un groupe phényle substitué par un atome de brome, et en particulier un groupe 3-bromophényle ou 4-bromophényle, tandis que $R^2$ représente un groupe méthyle, ou
- $R^1$ représente un groupe phénéthyle tandis que $R^2$ représente un atome d'hydrogène, ou
- $R^1$ représente un groupe carbazolyléthyle tandis que $R^2$ représente un atome d'hydrogène, ou
- $R^1$ représente un groupe 1,1-dioxotétra-hydrothién-3-yle tandis que $R^2$ représente un atome d'hydrogène, ou encore
- $R^1$ représente un groupe phénéthyle substitué par un groupe phénoxy, et en particulier un groupe 4-phénoxyphénéthyle, tandis que $R^2$ représente un atome d'hydrogène.

Lorsqu'ils ne sont pas disponibles commercialement, les azaboronates de formule générale (I) peuvent être synthétisés par réaction entre une bis(hydroxyalkyl)amine répondant à la formule générale (II) ci-après :

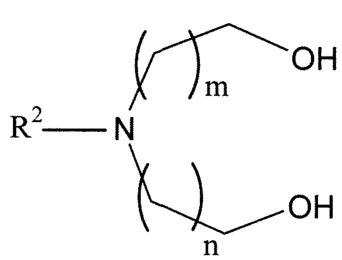

(II)

dans laquelle $R^2$, m et n ont la même signification que précédemment ;
et un composé boré répondant à la formule générale (III) ci-après :

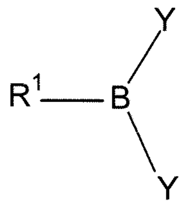

(III)

dans laquelle $R^2$ a la même signification que précédemment tandis que Y représente un atome ou un groupe apte à réagir avec un groupe hydroxyle pour former un groupe éther. Un tel atome ou groupe est, par exemple, un atome d'halogène, un groupe hydroxyle ou un groupe alcoxy.

Dans le cas où le composé boré de formule générale (III) est un acide boronique (Y = OH), alors la réaction entre ce composé et la bis(hydroxyalkyl)-amine de formule générale (II) est avantageusement réalisée en présence d'un agent de déshydratation tel qu'un tamis moléculaire, du sulfate de sodium anhydre ou du sulfate de magnésium anhydre, ou dans un appareil spécifique du type appareil de Dean-Stark.

Si la bis(hydroxyalkyl)amine de formule générale (II) n'est pas elle-même disponible commercialement, alors elle peut être obtenue par réaction entre une bis(hydroxyalkyl)amine répondant à la formule générale (IV) ci-après :

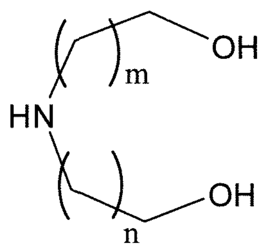

(IV)

dans laquelle m et n ont la même signification que précédemment ;
avec un composé de formule générale $R^2$-X dans laquelle X représente un atome partant tel qu'un atome d'halogène, ou un groupe partant tel qu'un groupe mésylate ou tosylate.

Conformément à l'invention, l'azaboronate de formule générale (I) est, de préférence, présent dans le capteur sous la forme d'un film mince qui recouvre l'une ou les deux faces d'un substrat convenablement choisi en fonction de la propriété physique dont les variations sont destinées à être mesurées par ce capteur.

En variante, l'azaboronate de formule générale (I) peut également être présent dans le capteur sous la forme d'un objet massif comme, par exemple, un cylindre présentant une certaine porosité de sorte à rendre accessible l'ensemble des molécules de cet azaboronate aux peroxydes.

Lorsque l'azaboronate de formule générale (I) se présente sous la forme d'un film mince, alors ce film a, de préférence, une épaisseur allant de 10 angstrӧms à 100 micromètres.

Un tel film peut notamment être obtenu par dépôt par pulvérisation, dépôt à la tournette (« *spin coating* » en langue anglo-saxonne), dépôt à la goutte, dépôt par jet d'encre ou encore par dépôt par sublimation, toutes ces techniques de dépôt étant bien connues de l'homme du métier.

Le substrat ainsi que le système de mesure du capteur sont choisis en fonction de la propriété physique de l'azaboronate de formule générale (I) dont les variations induites par la présence des peroxydes sont susceptibles d'être destinées à être mesurées par le capteur.

En l'espèce, les variations de masse des azaboronates de formule générale (I) et les variations de fluorescence de ces azaboronates, lorsqu'ils présentent des propriétés de fluorescence, se sont révélées particulièrement intéressantes à mesurer.

Aussi, le capteur est-il, de préférence, un capteur gravimétrique ou un capteur à fluorescence.

A titre d'exemples de capteurs gravimétriques, on peut citer les capteurs du type à microbalance à quartz, les capteurs à ondes de surface, plus connus sous la terminologie anglo-saxonne « *SAW* » pour « *Surface Acoustic Wave* », tels que les capteurs à ondes de Love et les capteurs à ondes de Lamb, ainsi que les microleviers.

Parmi les capteurs gravimétriques, on préfère plus particulièrement les capteurs à microbalance à quartz. Ce type de capteurs, dont le principe de fonctionnement a été décrit par J.A.O. Sanchez-Pedrono et al. dans Anal. Chem. Acta, vol. 182, 1986, 285, comprend, schématiquement, un substrat piézo-électrique (ou résonateur), généralement un cristal de quartz recouvert sur ses deux faces d'une couche métallique, par exemple d'or ou de platine, servant d'électrode. Le matériau sensible recouvrant l'une ou les deux faces du substrat, toute variation de masse de ce matériau se traduit par une variation de la fréquence de vibration du capteur.

Lorsque le capteur est un capteur à fluorescence, l'azaboronate de formule générale (I) comprend obligatoirement au moins un groupe fluorescent et ce groupe fluorescent est lié à l'atome d'azote de cet azaboronate, c'est-à-dire qu'il est représenté par $R^2$.

Conformément à l'invention, il est également possible d'utiliser un azaboronate de formule générale (I) comme matériau sensible dans des capteurs conçus pour mesurer des variations d'une propriété physique autre que la masse et la fluorescence comme, par exemple, des capteurs optiques basés sur la mesure de variations d'absorbance dans le domaine UV-visible ou encore de longueur d'onde dans le domaine des infrarouges.

Par ailleurs, il est également possible de réunir au sein d'un même dispositif ou « *multi-capteur* », plusieurs capteurs élémentaires comprenant des matériaux sensibles différents les uns des autres, ou munis de substrats et de systèmes de mesure différents les uns des autres comme, par exemple, un ou plusieurs capteurs gravimétriques et/ou un ou plusieurs capteurs à fluorescence, l'essentiel étant que l'un au moins de ces capteurs comprenne un azaboronate de formule générale (I).

Conformément à l'invention, les peroxydes destinés à être détectés par le capteur sont, de préférence, des peroxydes dont au moins l'un des deux atomes d'oxygène du groupe ou de l'un des groupes -O-O-qu'ils comportent est lié à un atome d'hydrogène, moyennant quoi cet atome d'oxygène forme un groupe hydroxyle avec cet atome d'hydrogène.

Des peroxydes qui répondent à ce critère sont notamment le peroxyde d'hydrogène, les hydro-peroxydes comme, par exemple, l'hydroperoxyde de *t*-butyle, l'hydroperoxyde d'$\alpha$-cumyle ou et l'hydro-peroxyde de 1-phénéthyle, et les peroxydes de cétones comme, par exemple, le peroxyde de méthyléthylcétone, le peroxyde d'acétylacétone ou le peroxyde de cyclo-hexanone, le peroxyde d'hydrogène étant particulièrement préféré.

Parmi les azaboronates répondant à la formule générale (I), certains sont connus et sont même disponibles commercialement tandis que d'autres semblent ne jamais avoir été décrits dans la littérature.

L'invention a donc de plus pour objet un azaboronate répondant à la formule générale (I) ci-avant, dans laquelle m et n valent 1 et :

- $R^1$ représente un groupe phényle tandis que $R^2$ représente un groupe anthracénylméthyle, ou
- $R^1$ représente un groupe benzofuryle, et en particulier un groupe 2-benzofuryle, tandis que $R^2$ représente un groupe anthracénylméthyle, ou
- $R^1$ représente un groupe phényle tandis que $R^2$ représente un groupe naphtylméthyle, ou encore
- $R^1$ représente un groupe phénéthyle tandis que $R^2$ représente un groupe éthyle.

L'invention a encore pour objet un capteur qui comprend au moins un azaboronate tel qu'il vient d'être défini, comme matériau sensible.

Là également, les particularités de ce capteur sont les mêmes que celles précédemment énoncées en relation avec l'utilisation d'un azaboronate de formule générale (I) comme matériau sensible dans un capteur destiné à détecter la présence d'un peroxyde dans un milieu gazeux.

D'autres caractéristiques et avantages de l'invention apparaîtront mieux à la lecture du complément de description qui suit, qui se rapporte à des exemples de synthèse d'azaboronates utiles selon l'invention, d'utilisation de ces azaboronates comme matériaux sensibles dans des capteurs et de démonstration des propriétés des capteurs ainsi obtenus.

Bien entendu, ces exemples ne sont donnés qu'à titre d'illustrations de l'objet de l'invention et ne constituent en aucun cas une limitation de cet objet.

## BRÈVE DESCRIPTION DES DESSINS

La figure 1 représente les variations de la fréquence de vibration des quartz de quatorze capteurs à microbalance à quartz comprenant chacun un film mince d'un azaboronate utile selon l'invention, telles qu'obtenues après 1 minute d'exposition à des vapeurs de peroxyde d'hydrogène.

Les figures 2, 3 et 4 représentent l'évolution de la fréquence de vibration du quartz de trois capteurs à microbalance à quartz comprenant chacun un film mince d'un azaboronate utile selon l'invention, telles qu'obtenue lorsque ces capteurs sont exposés successivement à l'air ambiant pendant 30 minutes, à des vapeurs de peroxyde d'hydrogène pendant 10 minutes, et à l'air ambiant pendant 20 minutes.

Les figures 5 et 6 représentent l'évolution de l'intensité du signal fluorescent émis par deux capteurs à fluorescence comprenant chacun un film mince d'un azaboronate utile selon l'invention, telle qu'obtenue lorsque ces capteurs sont exposés successivement à l'air ambiant pendant 30 minutes, à des vapeurs de peroxyde d'hydrogène pendant 10 minutes, et à l'air ambiant pendant 20 minutes.

La figure 7 représente les spectres d'émission de fluorescence d'un film mince d'un azaboronate utile selon l'invention, tels qu'obtenus avant d'exposer ce film mince à des vapeurs de peroxyde d'hydrogène (t0) et après avoir exposé ce film mince à des vapeurs de peroxyde d'hydrogène pendant 10 minutes.

La figure 8 représente l'évolution de l'intensité du signal fluorescent (courbe A) émis par un capteur à fluorescence comprenant un film mince d'un azaboronate utile selon l'invention, telle qu'obtenue lorsque ce capteur est exposé successivement à de l'air humide pendant 30 minutes, à des vapeurs de triperoxyde de triacétone (TATP) pendant 10 minutes, à de l'air humide pendant 20 minutes, à des vapeurs de TATP pendant 10 minutes, et à de l'air humide pendant 30 minutes ; sur cette figure, est également représentée la dérivée de l'intensité de fluorescence de ce signal en fonction du temps (courbe B).

## EXPOSÉ DÉTAILLÉ DE MODES DE RÉALISATION PARTICULIERS

**Exemple 1 : Synthèse d'azaboronates utiles selon l'invention :**

Le présent exemple se rapporte à la synthèse de différents azaboronates répondant à la formule générale (I).

### 1.1. Synthèse de l'azaboronate de formule générale (I) dans laquelle m = n = 1, $R^1$ = phényle et $R^2$ = éthyle :

Dans un montage de Dean-Stark, on introduit 135 mg (1 mmol) d'acide phénylboronique (société Aldrich, référence P20009), 112 mg (1 mmol) de N-éthyldiéthanolamine (société Aldrich, référence 112062) et 30 mL de toluène.

Après 48 heures de reflux, on laisse refroidir le milieu réactionnel jusqu'à ce qu'il soit à la température ambiante et on évapore le solvant.

On obtient ainsi 175 mg d'un solide jaune (soit un rendement de 80%) dont la caractérisation RMN du proton est donnée ci-après.

**RMN $^1$H (200 MHz, $CDCl_3$) $\delta$ ppm** : 1,06 (t, 3H), 2,38 (d, 2H), 2,95 (m, 4H), 4,14 (m, 4H), 7,25 (m, 3H), 7,56 (m, 2H)

### 1.2. Synthèse de l'azaboronate de formule générale (I) dans laquelle m = n = 1, $R^1$ = phényle et $R^2$ = anthracénylméthyle :

* *Synthèse de la N-anthracénylméthyldiéthanolamine :*

Dans un tricol de 250 mL muni d'un réfrigérant, on introduit sous argon 5 g (22 mmol) de chlorométhylanthracène (société Aldrich, référence 196517), 4,7 g (44 mmol) de diéthanolamine (société Aldrich, référence D8885), 3,6 g (26 mmol) de carbonate de potassium, 100 mg (0,6 mmol) d'iodure de potassium et 150 mL d'acétonitrile.

Après 5 heures de reflux, on laisse refroidir le milieu réactionnel jusqu'à ce qu'il soit à la température ambiante puis on le verse sur 500 mL d'eau. On filtre le solide ainsi obtenu et on le lave à l'eau.

On obtient ainsi 5,8 g d'un solide jaune (soit un rendement de 89%) dont la caractérisation RMN du proton est donnée ci-après.

**RMN $^1$H (200 MHz, $CDCl_3$) $\delta$ ppm** : 2,43 (s, 2H), 2,65 (t, 4H), 3,5 (t, 4H), 4,68 (2H), 7,3-7,6 (m, 4H), 8 (d, 2H), 8,45 (d, 3H)

* *Synthèse de l'azaboronate :*

Dans un montage de Dean-Stark, on introduit 118 mg (1 mmol) d'acide phénylboronique (société Aldrich, référence P20009), 304 mg (1 mmol) de N-anthracénylméthyldiéthanolamine et 30 mL de toluène.

Après 24 heures de reflux, on laisse refroidir le milieu réactionnel jusqu'à ce qu'il soit à la température ambiante et on filtre le solide obtenu.

On obtient ainsi 298 mg d'une poudre jaune (soit un rendement de 78%) dont la caractérisation RMN du proton est donnée ci-après.

**RMN $^1$H (200 MHz, MeOD) $\delta$ ppm** : 2,81 (t, 4H), 3,56 (t, 4H), 4,74 (s, 2H), 7,3-7,6 (m, 9H), 8 (d, 2H), 8,5 (s, 1H), 8,6 (d, 2H)

### 1.3. Synthèse de l'azaboronate de formule générale (I) dans laquelle m = n = 1, $R^1$ = 2-benzofuryle et $R^2$ = anthracénylméthyle :

Dans un montage de Dean-Stark, on introduit 97 mg (6,5 mmol) d'acide 2-benzofurylboronique (société Aldrich, référence 499943), 201 mg (6,5 mmol) de N-anthracénylméthyldiéthanolamine telle qu'obtenue au point 1.2 ci-avant et 25 mL de toluène.

Après 24 heures de reflux, on laisse refroidir le milieu réactionnel jusqu'à ce qu'il soit à la température ambiante et on filtre le solide obtenu.

On obtient ainsi 62 mg d'une poudre jaune (soit un rendement de 23%) dont la caractérisation RMN du proton est donnée ci-après.
**RMN $^1$H (200 MHz, MeOD)** $\delta$ **ppm** : 2,95 (s, 4H), 3,62 (t, 4H), 4,9 (d, 2H), 7,16 (m, 2H), 7,5 (m, 6H), 8,02 (d, 2H), 8,54 (S, 1H), 8,59 (s, 2H)

**1.4. Synthèse de l'azaboronate de formule générale (I) dans laquelle m = n = 1, $R^1$ = phényle et $R^2$ = naphtylméthyle :**

* *Synthèse de la N-naphtylméthyldiéthanolamine :*

Dans un tricol de 100 mL muni d'un réfrigérant, on introduit sous argon 1,76 g (10 mmol) de chlorométhylnaphtalène (société Aldrich, référence 25170), 2,11 g (20 mmol) de diéthanolamine (société Aldrich, référence D8885), 1,65 g (12 mmol) de carbonate de potassium, 17 mg (0,3 mmol) d'iodure de potassium et 35 mL d'acétonitrile.
Après 5 heures de reflux, on laisse refroidir le milieu réactionnel jusqu'à ce qu'il soit à la température ambiante puis on le verse sur 500 mL d'eau. On filtre le solide ainsi obtenu et on le lave à l'eau.
On obtient ainsi 2 g d'un solide crème (soit un rendement de 82%) dont la caractérisation RMN du proton est donnée ci-après.
**RMN $^1$H (200 MHz, $CDCl_3$)** $\delta$ **ppm** : 2,71 (t, 4H), 3,29 (s, 2H), 3,51 (t, 4H), 4,1 (s, 2H), 7,5 (m, 4H), 7,8 (m, 2H), 8,26 (d, 1H)

* *Synthèse de l'azaboronate :*

Dans un montage de Dean-Stark, on introduit 246 mg (2 mmol) d'acide phénylboronique (société Aldrich, référence P20009), 500 mg (2 mmol) de *N*-naphtylméthyldiéthanolamine et 30 mL de toluène.
Après 24 heures de reflux, on laisse refroidir le milieu réactionnel jusqu'à ce qu'il soit à la température ambiante et on filtre le solide obtenu.
On obtient ainsi 280 mg d'une poudre blanche (soit un rendement de 42%) dont la caractérisation RMN du proton est donnée ci-après.
**RMN $^1$H (200 MHz, $CDCl_3$)** $\delta$ **ppm :** 3, 1 (s, 4H), 3,95 (s, 2H), 4,25 (t, 4H), 7,5 (m, 8H), 7,84 (m, 6H)

**1.5. Synthèse de l'azaboronate de formule générale (I) dans laquelle m = n = 1, $R^1$ = phénéthyle et $R^2$ = méthyle :**

Dans un montage de Dean-Stark, on introduit 135 mg (2 mmol) d'acide phénéthylboronique (société Aldrich, référence 588423), 122 mg (2 mmol) de N-éthyldiéthanolamine (société Aldrich, référence 112062) et 30 mL de toluène.
Après 24 heures de reflux, on laisse refroidir le milieu réactionnel jusqu'à ce qu'il soit à la température ambiante et on évapore le solvant.
On obtient ainsi 392 mg d'un solide jaune (soit un rendement de 79%) dont la caractérisation RMN du proton est donnée ci-après.
**RMN $^1$H (200 MHz, $CDCl_3$)** $\delta$ **ppm** : 0,72 (m, 2H), 1,23 (t, 3H), 2,69 (m, 2H), 2,88 (m, 6H), 3,94 (m, 4H), 7,14 (m, 5H)

**Exemple 2 : Tests de détection par gravimétrie :**

L'aptitude des azaboronates de formule générale (I) à servir de matériaux sensibles dans des capteurs gravimétriques destinés à détecter la présence de peroxydes dans un milieu gazeux est vérifiée par une série de tests dans lesquels sont utilisés :

- des capteurs à microbalance à quartz, comprenant un quartz de coupe AT, de fréquence de vibration de 9 MHz, muni de deux électrodes de mesure circulaires en or (société AMETEK PRECISION INSTRUMENTS, modèle QA9RA-50), comme capteurs gravimétriques ; et
- les azaboronates 1 à 14 répertoriés dans le tableau 1 ci-après, comme matériaux sensibles ; et
- le peroxyde d'hydrogène comme peroxyde.

Tableau 1

| Azaboronate de formule générale (I) | m | n | $R^1$ | $R^2$ |
|---|---|---|---|---|
| 1 | 1 | 1 | phényle | éthyle |
| 2 | 1 | 1 | 3-bromophényle | n-butyle |

(suite)

| Azaboronate de formule générale (I) | m | n | $R^1$ | $R^2$ |
|---|---|---|---|---|
| **3** | 1 | 1 | 4-bromophényle | n-butyle |
| **4** | 1 | 1 | phényle | anthracénylméthyle |
| **5** | 1 | 1 | 2-benzofuryle | anthracénylméthyle |
| **6** | 1 | 1 | phényle | n-butyle |
| **7** | 1 | 1 | pyridyle | phényle |
| **8** | 1 | 1 | phényle | naphtylméthyle |
| **9** | 1 | 1 | phénéthyle | éthyle |
| **10** | 1 | 1 | 3-bromophényle | méthyle |
| **11** | 1 | 1 | phénéthyle | H |
| **12** | 1 | 1 | carbazolyléthyle | H |
| **13** | 1 | 1 | 1,1-dioxotétrahydrothién-3-yle | H |
| **14** | 1 | 1 | 4-phénoxyphénéthyle | H |

Les azaboronates 1, 4, 5, 8 et 9 correspondent aux 5 azaboronates dont la synthèse est décrite dans l'exemple 1 ci-avant, tandis que les azaboronates 2, 3, 6, 7 et 10 à 14 sont des composés disponibles, pour les 4 premiers, auprès de la société Aldrich sous les références 680486, 680494, 680478 et 647284, et, pour les 5 derniers, auprès de la société Alfa Aesar sous les références L17796, L19706, L19575, H31355 et H31401.

Pour les besoins des tests, on dépose, sur l'une des faces de chacun des quartz, un film mince de l'un des azaboronates 1 à 14. Ces dépôts sont réalisés soit par la technique de dépôt à la tournette soit par pulvérisation à partir de solutions contenant de 4 à 7 mg/mL d'azaboronate dans le chloroforme.

La formation des films se traduit par une perte de fréquence de la vibration du quartz des capteurs allant de 3 à 7 kHz.

Les capteurs sont ensuite exposés à l'air ambiant pendant 30 minutes, puis à du peroxyde d'hydrogène, à une concentration proche de la pression de vapeur de ce peroxyde, soit environ 1500 ppm, dans de l'air ambiant, pendant 10 minutes, et de nouveau à l'air ambiant pendant 20 minutes.

La sensibilité des capteurs au peroxyde d'hydrogène est appréciée en déterminant, pour chacun d'eux, la variation ($\Delta F$) de la fréquence de vibration du quartz obtenue après 1 minute d'exposition au peroxyde d'hydrogène, cette variation étant déterminée comme suit :

$\Delta F$ = moyenne des fréquences mesurées avant l'exposition au $H_2O_2$ – fréquence de vibration au temps $t_{1min}$ de l'exposition au $H_2O_2$.

Les résultats sont illustrés sur la figure 1 sous la forme d'un graphique indiquant les valeurs de $\Delta F$, exprimées en hertz (Hz), telles qu'obtenues pour chacun des capteurs. Sur cette figure, les capteurs sont numérotés de 1 à 14 et portent le même numéro que celui de l'azaboronate dont leur quartz est recouvert.

Cette figure montre que tous les capteurs testés ont réagi à la présence du peroxyde d'hydrogène dès la première minute d'exposition à ce peroxyde puisqu'une chute de la fréquence de vibration du quartz, plus ou moins prononcée mais toujours significative est observée pour chacun d'eux. A cet égard, il convient, en effet, de préciser que, dans le domaine des capteurs à microbalance à quartz, une variation de la fréquence de vibration du quartz d'un capteur est considérée comme significative et, donc, exploitable dès lors qu'elle est supérieure à trois fois le bruit de fond de ce capteur, soit environ 10 Hz dans le cas présent. Or, comme visible sur la figure 1, la chute de la fréquence de vibration du quartz des 14 capteurs testés est très largement supérieure à cette valeur seuil.

Par ailleurs, les figures 2, 3 et 4 illustrent l'évolution de la fréquence de vibration du quartz des capteurs comprenant respectivement les azaboronates 2, 3 et 4 comme matériau sensible, telle qu'observée pendant toute la durée des expositions de ces capteurs à l'air ambiant et au peroxyde d'hydrogène, sous la forme d'une courbe représentant les valeurs de la fréquence de vibration du quartz, exprimées en Hz, en fonction du temps, exprimé en minutes.

Ces figures confirment que la chute de la fréquence de vibration du quartz des capteurs est bien induite par la présence du peroxyde d'hydrogène et commence à se produire dès la mise en contact des capteurs avec ce peroxyde.
Cette chute se poursuit ensuite pour atteindre la valeur de 2100 Hz dans le cas des capteurs ayant les azaboronates 2 et 3 comme matériau sensible, et celle de 3000 Hz pour le capteur ayant l'azaboronate 4 comme matériau sensible.
Cette chute est réversible puisque la remise des capteurs au contact de l'air ambiant se traduit par une remontée de la fréquence de vibration du quartz, rapide dans le cas des capteurs ayant les azaboronates 2 et 3 comme matériau sensible, plus lente dans le cas du capteur ayant l'azaboronate 4 comme matériau sensible.

**<u>Exemple 3</u> : <u>Tests de détection par fluorescence</u> :**

Pour vérifier l'aptitude des azaboronates de formule générale (I), dans laquelle $R^2$ représente un groupe fluorescent, à servir de matériaux sensibles dans des capteurs à fluorescence destinés à détecter la présence de peroxydes et, en particulier du peroxyde d'hydrogène, dans un milieu gazeux, un premier test est réalisé en utilisant les azaboronates respectivement numérotés 4 et 5 dans le tableau 1 ci-avant.
Pour les besoins de ces tests, on dépose, sur l'une des faces de deux substrats en verre du type lames de microscope (75 mm x 25 mm x 1 mm, Heathrow Scientific), un film mince de l'un de ces azaboronates par la technique de dépôt à la tournette à partir d'une solution contenant 5 mg/mL de l'azaboronate 4 dans le chloroforme.
Ces capteurs sont alors exposés à l'air ambiant pendant 30 minutes, puis à du peroxyde d'hydrogène, à une concentration proche de la pression de vapeur de ce peroxyde, soit environ 1500 ppm, dans de l'air ambiant, pendant 10 minutes, puis de nouveau à l'air ambiant pendant 20 minutes.
Les figures 5 et 6 illustrent l'évolution du signal fluorescent émis par ces capteurs au cours de ces expositions, sous la forme de courbes représentant l'intensité de ce signal, exprimée en volts, en fonction du temps, exprimé en minutes ($\lambda_{excitation}$ : 300-380 nm ; $\lambda_{émission}$ : > 420 nm).
La figure 5 correspond au capteur ayant l'azaboronate 4 comme matériau sensible tandis que la figure 6 correspond au capteur ayant l'azaboronate 5 comme matériau sensible.
Ces figures montrent que les deux capteurs ont réagi à la présence du peroxyde d'hydrogène et ce, dès leur mise en contact avec ce peroxyde, mais qu'ils ont réagi de manière différente puisque l'on observe une chute de l'intensité du signal fluorescent émis par le capteur ayant l'azaboronate 4 comme matériau sensible alors que l'on observe au contraire une augmentation de l'intensité du signal fluorescent émis par le capteur ayant l'azaboronate 5 comme matériau sensible.
Dans les deux cas, la modification de l'intensité du signal fluorescent est suffisamment prononcée pour être exploitable.
Un deuxième test est réalisé :

- en déposant sur l'une des faces d'un substrat en quartz un film mince de l'azaboronate numéroté 8 dans le tableau 1 ci-avant, par la technique de dépôt à la tournette à partir d'une solution contenant 5 mg/mL de cet azaboronate dans le chloroforme ;
- en mesurant le spectre d'émission de fluorescence ($\lambda_{excitation}$ : 287 nm) du capteur ainsi obtenu (t0) ;
- en exposant ce capteur à du peroxyde d'hydrogène, à une concentration proche de la pression de vapeur de ce peroxyde, soit environ 1500 ppm, dans de l'air ambiant, pendant 10 minutes ; et
- en remesurant le spectre d'émission de fluorescence du capteur au terme de cette exposition (t10).

La figure 7 représente les spectres d'émission de fluorescence du capteur tels que respectivement obtenus à t0 et t10.
Comme le montre cette figure, le capteur a réagi très fortement à la présence du peroxyde d'hydrogène puisque sa mise en contact avec ce peroxyde s'est traduite par une extinction de la fluorescence.
Un troisième test est réalisé :

- en déposant sur l'une des faces d'un substrat en quartz un film mince de l'azaboronate numéroté 4 dans le tableau 1 ci-avant, par la technique de dépôt à la tournette à partir d'une solution contenant 5 mg/mL de cet azaboronate dans le chloroforme ;
- en exposant le capteur ainsi obtenu à de l'air humide pendant 30 minutes, puis à du triperoxyde de triacétone (TATP), à une concentration de l'ordre de 20 ppm, dans de l'air humide pendant 10 minutes, puis de nouveau à de

l'air humide pendant 20 minutes, puis de nouveau à du TATP, également à une concentration de l'ordre de 20 ppm, dans de l'air humide pendant 10 minutes, et enfin à de l'air humide pendant 30 minutes ; et

- en suivant l'évolution de l'intensité du signal fluorescent émis par le capteur au cours de ces expositions.

Cette évolution est illustrée sur la figure 8, sous la forme d'une courbe (courbe A) représentant l'intensité du signal fluorescent, exprimée en volts, en fonction du temps, exprimé en minutes ($\lambda_{excitation}$ : 300-380 nm ; $\lambda_{émission}$ : > 420 nm). Sur la figure 8, est également représentée la dérivée de l'intensité du signal fluorescent en fonction du temps (courbe B).

Cette figure montre que le capteur a bien réagi à la présence du TATP et ce, dès sa mise en contact avec ce peroxyde. Les effets de la présence du TATP sur le signal de fluorescence émis par le capteur sont particulièrement bien visibles sur la courbe B de cette figure.

## Revendications

1. Utilisation d'un azaboronate répondant à la formule générale (I) ci-après :

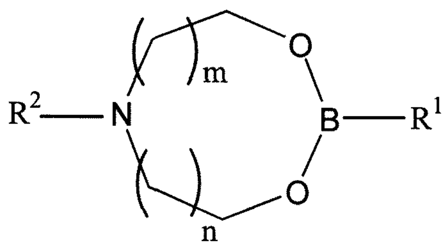

(I)

dans laquelle :

m et n valent, indépendamment l'un de l'autre, 1 ou 2 ;
$R^1$ représente un groupe alkyle comprenant de 1 à 6 atomes de carbone, un groupe hétérocycloalkyle comprenant de 1 à 3 cycles à 5 ou 6 chaînons chacun, un groupe aryle ou hétéroaryle comprenant de 1 à 3 cycles à 5 ou 6 chaînons chacun, un groupe arylalkyle ou hétéroarylalkyle dont le radical alkyle comprend de 1 à 6 atomes de carbone et dont le radical aryle ou hétéroaryle comprend de 1 à 3 cycles à 5 ou 6 chaînons chacun ; tandis que
$R^2$ représente un atome d'hydrogène ou un groupe alkyle comprenant de 1 à 6 atomes de carbone, un groupe hétérocycloalkyle comprenant de 1 à 3 cycles à 5 ou 6 chaînons chacun, un groupe aryle ou hétéroaryle comprenant de 1 à 3 cycles à 5 ou 6 chaînons chacun, un groupe arylalkyle ou hétéroarylalkyle dont le radical alkyle comprend de 1 à 6 atomes de carbone et dont le radical aryle ou hétéroaryle comprend de 1 à 3 cycles à 5 ou 6 chaînons chacun ;

comme matériau sensible dans un capteur destiné à détecter la présence d'un peroxyde dans un milieu gazeux par mise en contact de ce capteur avec ce milieu gazeux.

2. Utilisation selon la revendication 1, dans laquelle $R^1$ représente un groupe phényle, un groupe phényle substitué par un ou plusieurs atomes d'halogène, un groupe benzofuryle, un groupe phénéthyle, un groupe phénéthyle substitué par un groupe phénoxy, un groupe pyridyle, un groupe carbazolyléthyle, un groupe tétrahydrothiényle ou un groupe tétrahydrothiényle dont l'atome de soufre est lié à deux atomes d'oxygène.

3. Utilisation selon la revendication 1, dans laquelle $R^2$ représente un atome d'hydrogène, un groupe alkyle comprenant de 1 à 4 atomes de carbone, un groupe phényle, un groupe naphtylméthyle ou un groupe anthracénylméthyle.

4. Utilisation selon l'une quelconque des revendications précédentes, dans laquelle m et n valent 1.

5. Utilisation selon la revendication 1, dans laquelle l'azaboronate répond à la formule générale (I) dans laquelle m et n valent 1 et dans laquelle :

- $R^1$ représente un groupe phényle tandis que $R^2$ représente un groupe éthyle, ou

- $R^1$ représente un groupe phényle substitué par un atome de brome, tandis que $R^2$ représente un groupe n-butyle, ou
- $R^1$ représente un groupe phényle tandis que $R^2$ représente un groupe anthracénylméthyle, ou
- $R^1$ représente un groupe benzofuryle tandis que $R^2$ représente un groupe anthracénylméthyle, ou
- $R^1$ représente un groupe phényle tandis que $R^2$ représente un groupe n-butyle, ou
- $R^1$ représente un groupe pyridyle tandis que $R^2$ représente un groupe phényle, ou
- $R^1$ représente un groupe phényle tandis que $R^2$ représente un groupe naphtylméthyle, ou
- $R^1$ représente un groupe phénéthyle tandis que $R^2$ représente un groupe éthyle, ou
- $R^1$ représente un groupe phényle substitué par un atome de brome, tandis que $R^2$ représente un groupe méthyle, ou
- $R^1$ représente un groupe phénéthyle tandis que $R^2$ représente un atome d'hydrogène, ou
- $R^1$ représente un groupe carbazolyléthyle tandis que $R^2$ représente un atome d'hydrogène, ou
- $R^1$ représente un groupe 1,1-dioxotétrahydrothién-3-yle tandis que $R^2$ représente un atome d'hydrogène, ou encore
- $R^1$ représente un groupe phénéthyle substitué par un groupe phénoxy tandis que $R^2$ représente un atome d'hydrogène.

**6.** Utilisation selon l'une quelconque des revendications précédentes, dans laquelle l'azaboronate est présent dans le capteur sous la forme d'un film mince recouvrant l'une ou les deux faces d'un substrat.

**7.** Utilisation selon l'une quelconque des revendications précédentes, dans laquelle le capteur est un capteur gravimétrique, de préférence un capteur à microbalance à quartz.

**8.** Utilisation selon l'une quelconque des revendications 1 à 6, dans laquelle le capteur est un capteur à fluorescence.

**9.** Utilisation selon l'une quelconque des revendications précédentes, dans laquelle le peroxyde est choisi parmi le peroxyde d'hydrogène, les hydroperoxydes et les peroxydes de cétones.

**10.** Utilisation selon la revendication 9, dans laquelle le peroxyde est le peroxyde d'hydrogène.

**11.** Azaboronate répondant à la formule générale (I) ci-après :

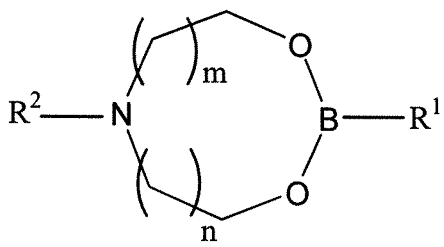

(I)

dans laquelle m et n valent 1 et :

- $R^1$ représente un groupe phényle tandis que $R^2$ représente un groupe anthracénylméthyle, ou
- $R^1$ représente un groupe benzofuryle, et en particulier un groupe 2-benzofuryle, tandis que $R^2$ représente un groupe anthracénylméthyle, ou
- $R^1$ représente un groupe phényle tandis que $R^2$ représente un groupe naphtylméthyle, ou encore
- $R^1$ représente un groupe phénéthyle tandis que $R^2$ représente un groupe éthyle.

**12.** Capteur, qui comprend un azaboronate selon la revendication 11 comme matériau sensible.

**13.** Capteur selon la revendication 12, qui est un capteur gravimétrique, de préférence un capteur à microbalance à quartz.

**14.** Capteur selon la revendication 12, qui est un capteur à fluorescence.

**15.** Capteur selon l'une quelconque des revendications 12 à 14, qui est destiné à détecter le peroxyde d'hydrogène.

**Patentansprüche**

1. Verwendung eines Azaboronats, das der nachfolgenden allgemeinen Formel (I) genügt:

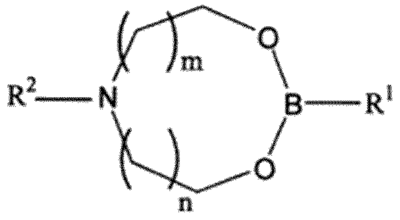

(I)

wobei:

m und n unabhängig voneinander den Wert 1 oder 2 haben;
$R^1$ eine Alkylgruppe mit 1 bis 6 Kohlenstoffatomen repräsentiert, eine Heterocycloalkylgruppe mit 1 bis 3 Ringen mit jeweils 5 oder 6 Gliedern, eine Aryl- oder Heteroarylgruppe mit 1 bis 3 Ringen mit jeweils 5 oder 6 Gliedern, eine Arylalkyl- oder Heteroarylalkylgruppe, deren Alkylradikal 1 bis 6 Kohlenstoffatome umfasst und deren Aryl- oder Heteroarylradikal 1 bis 3 Ringe mit jeweils 5 oder 6 Gliedern umfasst; wohingegen
$R^2$ ein Wasserstoffatom repräsentiert oder eine Alkylgruppe mit 1 bis 6 Kohlenstoffatomen, eine Heterocycloalkylgruppe mit 1 bis 3 Ringen mit jeweils 5 oder 6 Gliedern, eine Aryl- oder Heteroarylgruppe mit 1 bis 3 Ringen mit jeweils 5 oder 6 Gliedern, eine Arylalkyl- oder Heteroarylalkylgruppe, deren Alkylradikal 1 bis 6 Kohlenstoffatome umfasst und deren Aryl- oder Heteroarylradikal 1 bis 3 Ringe mit jeweils 5 oder 6 Gliedern umfasst;

als sensibles Material in einem Sensor, der zur Erfassung des Vorhandenseins eines Peroxyds in einer gasförmigen Umgebung durch Inkontaktbringen dieses Sensors mit dieser gasförmigen Umgebung ausgelegt ist.

2. Verwendung nach Anspruch 1, wobei $R^1$ eine Phenylgruppe repräsentiert, eine Phenylgruppe, die mit einem oder mehreren Halogenatomen substituiert ist, eine Benzofurylgruppe, eine Phenethylgruppe, eine Phenethylgruppe, die mit einer Phenoxygruppe substituiert ist, eine Pyridylgruppe, eine Carbazolylethylgruppe, eine Tetrahydrothienylgruppe oder eine Tetrahydrothienylgruppe, deren Schwefelatom an zwei Sauerstoffatome gebunden ist.

3. Verwendung nach Anspruch 1, wobei $R^2$ ein Wasserstoffatom repräsentiert, eine Alkylgruppe mit 1 bis 4 Kohlenstoffatomen, eine Phenylgruppe, eine Naphtylmethylgruppe oder eine Antracenylmethylgruppe.

4. Verwendung nach einem der vorhergehenden Ansprüche, wobei der Wert von m und n 1 ist.

5. Verwendung nach Anspruch 1, wobei das Azaboronat der allgemeinen Formel (I) genügt, wobei m und n den Wert 1 haben, und wobei:

- $R^1$ eine Phenylgruppe repräsentiert, wohingegen $R^2$ eine Ethylgruppe repräsentiert, oder
- $R^1$ eine Phenylgruppe repräsentiert, die mit einem Bromatom substituiert ist, wohingegen $R^2$ eine n-Butylgruppe repräsentiert, oder
- $R^1$ eine Phenylgruppe repräsentiert, wohingegen $R^2$ eine Anthracenylmethylgruppe repräsentiert, oder
- $R^1$ eine Benzofurylgruppe repräsentiert, wohingegen $R^2$ eine Anthracenylmethylgruppe repräsentiert, oder
- $R^1$ eine Phenylgruppe repräsentiert, wohingegen $R^2$ eine n-Butylgruppe repräsentiert, oder
- $R^1$ eine Pyridylgruppe repräsentiert, wohingegen $R^2$ eine Phenylgruppe repräsentiert, oder
- $R^1$ eine Phenylgruppe repräsentiert, wohingegen $R^2$ eine Naphtylmethylgruppe repräsentiert, oder
- $R^1$ eine Phenethylgruppe repräsentiert, wohingegen $R^2$ eine Ethylgruppe repräsentiert, oder
- $R^1$ eine Phenylgruppe repräsentiert, die mit einem Bromatom substituiert ist, wohingegen $R^2$ eine Methylgruppe repräsentiert, oder
- $R^1$ eine Phenethylgruppe repräsentiert, wohingegen $R^2$ ein Wasserstoffatom repräsentiert, oder
- $R^1$ eine Carbazolylethylgruppe repräsentiert, wohingegen $R^2$ ein Wasserstoffatom repräsentiert, oder
- $R^1$ eine Gruppe 1,1-Dioxotetra-Hydrothien-3-yl repräsentiert, wohingegen $R^2$ ein Wasserstoffatom repräsentiert, oder aber
- $R^1$ eine Phenethylgruppe repräsentiert, die mit einer Phenoxygruppe substituiert ist, wohingegen $R^2$ ein Wasserstoffatom repräsentiert.

6. Verwendung nach einem der vorhergehenden Ansprüche, wobei das Azaboronat in dem Sensor in Form eines dünnen Films vorhanden ist, der eine oder beide Flächen eines Substrats bedeckt.

7. Verwendung nach einem der vorhergehenden Ansprüche, wobei der Sensor ein gravimetrischer Sensor ist, vorzugsweise ein Quarzmikrowaagensensor.

8. Verwendung nach einem der Ansprüche 1 bis 6, wobei der Sensor ein Fluoreszenzsensor ist.

9. Verwendung nach einem der vorhergehenden Ansprüche, wobei das Peroxyd ausgewählt ist aus Wasserstoffperoxyd, den Hydro-Peroxyden und den Keton-Peroxyden.

10. Verwendung nach Anspruch 9, wobei das Peroxyd Wasserstoffperoxyd ist.

11. Azaboronat, das der nachfolgenden allgemeinen Formel (I) genügt:

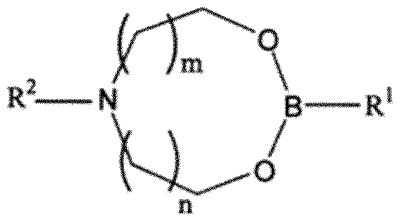

(I)

wobei m und n den Wert 1 haben, und wobei:

- $R^1$ eine Phenylgruppe repräsentiert, wohingegen $R^2$ eine Anthracenylmethylgruppe repräsentiert, oder
- $R^1$ eine Benzofurylgruppe repräsentiert, und insbesondere eine Gruppe 2-Benzofuryl, wohingegen $R^2$ eine Anthracenylmethylgruppe repräsentiert, oder
- $R^1$ eine Phenylgruppe repräsentiert, wohingegen $R^2$ eine Naphtylmethylgruppe repräsentiert, oder aber
- $R^1$ eine Phenethylgruppe repräsentiert, wohingegen $R^2$ eine Ethylgruppe repräsentiert.

12. Sensor, der ein Azaboronat nach Anspruch 11 als sensibles Material umfasst.

13. Sensor nach Anspruch 12, der ein gravimetrischer Sensor ist, vorzugsweise ein Quarzmikrowaagensensor.

14. Sensor nach Anspruch 12, der ein Fluoreszenzsensor ist.

15. Sensor nach einem der Ansprüche 12 bis 14, der dazu ausgelegt ist, Wasserstoffperoxyd zu erfassen.

**Claims**

1. Use of an azaboronate corresponding to general formula (I) below:

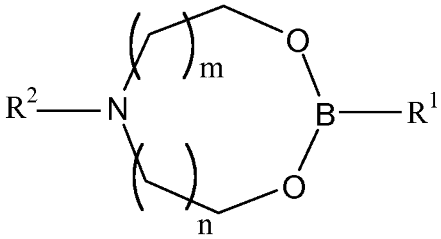

(I)

in which:

m and n independently are 1 or 2;
$R^1$ represents an alkyl group comprising 1 to 6 carbon atoms, a heterocycloalkyl group comprising 1 to 3 cycles

with 5 or 6 members each, an aryl or heteroaryl group comprising 1 to 3 cycles with 5 or 6 members each, an arylalkyl or heteroarylalkyl group in which the alkyl radical comprises 1 to 6 carbon atoms, and in which the aryl or heteroaryl radical comprises 1 to 3 cycles with 5 or 6 members each; whilst
$R^2$ represents a hydrogen atom or an alkyl group comprising 1 to 6 carbon atoms, a heterocycloalkyl group comprising 1 to 3 cycles with 5 or 6 members each, an aryl or heteroaryl group comprising 1 to 3 cycles with 5 or 6 members each, an arylalkyl or heteroarylalkyl group in which the alkyl radical comprises 1 to 6 carbon atoms, and in which the aryl or heteroaryl radical comprises 1 to 3 cycles with 5 or 6 members each;

as a sensitive material in a sensor for the detection of the presence of a peroxide in a gaseous environment by placing the sensor in contact with the gaseous environment.

**2.** Use according to claim 1, in which $R^1$ represents a phenyl group, a phenyl group substituted by one or more halogen atoms, a benzofuryl group, a phenethyl group, a phenethyl group substituted by a phenoxy group, a pyridyl group, a carbazolylethyl group, a tetrahydrothienyl group, or a tetrahydrothienyl group in which the sulphur atom is bonded to two oxygen atoms.

**3.** Use according to claim 1, in which $R^2$ represents a hydrogen atom, an alkyl group comprising 1 to 4 carbon atoms, a phenyl group, a naphthylmethyl group, or an anthracenylmethyl group.

**4.** Use according to any of the preceding claims, in which m and n are 1.

**5.** Use according to claim 1, in which the azaboronate corresponds to general formula (I), in which m and n are 1, and in which:

- $R^1$ represents a phenyl group, whilst $R^2$ represents an ethyl group, or
- $R^1$ represents a phenyl group substituted by a bromine atom, whilst $R^2$ represents an *n*-butyl group, or
- $R^1$ represents a phenyl group, whilst $R^2$ represents an anthracenyl-methyl group, or
- $R^1$ represents a benzofuryl group, whilst $R^2$ represents an anthracenylmethyl group, or
- $R^1$ represents a phenyl group, whilst $R^2$ represents an *n*-butyl group, or
- $R^1$ represents a pyridyl group, whilst $R^2$ represents a phenyl group, or
- $R^1$ represents a phenyl group, whilst $R^2$ represents a naphthylmethyl group, or
- $R^1$ represents a phenethyl group, whilst $R^2$ represents an ethyl group, or
- $R^1$ represents a phenyl group substituted by a bromine atom, whilst $R^2$ represents a methyl group, or
- $R^1$ represents a phenethyl group, whilst $R^2$ represents a hydrogen atom, or
- $R^1$ represents a carbazolylethyl group, whilst $R^2$ represents a hydrogen atom, or
- $R^1$ represents a 1,1-dioxotetrahydrothien-3-yl group, whilst $R^2$ represents a hydrogen atom, or still
- $R^1$ represents a phenethyl group substituted by a phenoxy group, whilst $R^2$ represents a hydrogen atom.

**6.** Use according to any of the preceding claims, in which the azaboronate is present in the sensor in the form of a thin film covering one or both surfaces of a substrate.

**7.** Use according to any of the preceding claims, in which the sensor is a gravimetric sensor, preferably a quartz microbalance sensor.

**8.** Use according to any of claims 1 to 6, in which the sensor is a fluorescence sensor.

**9.** Use according to any of the preceding claims, in which the peroxide is chosen from hydrogen peroxide, hydroperoxides, and ketone peroxides.

**10.** Use according to claim 9, in which the peroxide is hydrogen peroxide.

**11.** Azaboronate corresponding to general formula (I) below:

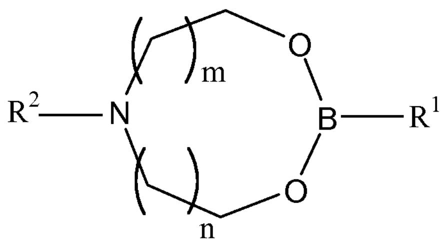

(I)

in which m and n equal 1 and:

- $R^1$ represents a phenyl group, whilst $R^2$ represents an anthracenyl-methyl group; or
- $R^1$ represents a benzofuryl group, and, in particular, a 2-benzofuryl group, whilst $R^2$ represents an anthracenylmethyl group, or
- $R^1$ represents a phenyl group, whilst $R^2$ represents a naphthylmethyl group, or still
- $R^1$ represents a phenethyl group, whilst $R^2$ represents an ethyl group.

**12.** Sensor comprising an azaboronate according to claim 11 as a sensitive material.

**13.** Sensor according to claim 12, which is a gravimetric sensor, preferably a quartz microbalance sensor.

**14.** Sensor according to claim 12, which is a fluorescence sensor.

**15.** Sensor according to any of claims 12 to 14, which is intended to detect hydrogen peroxide.

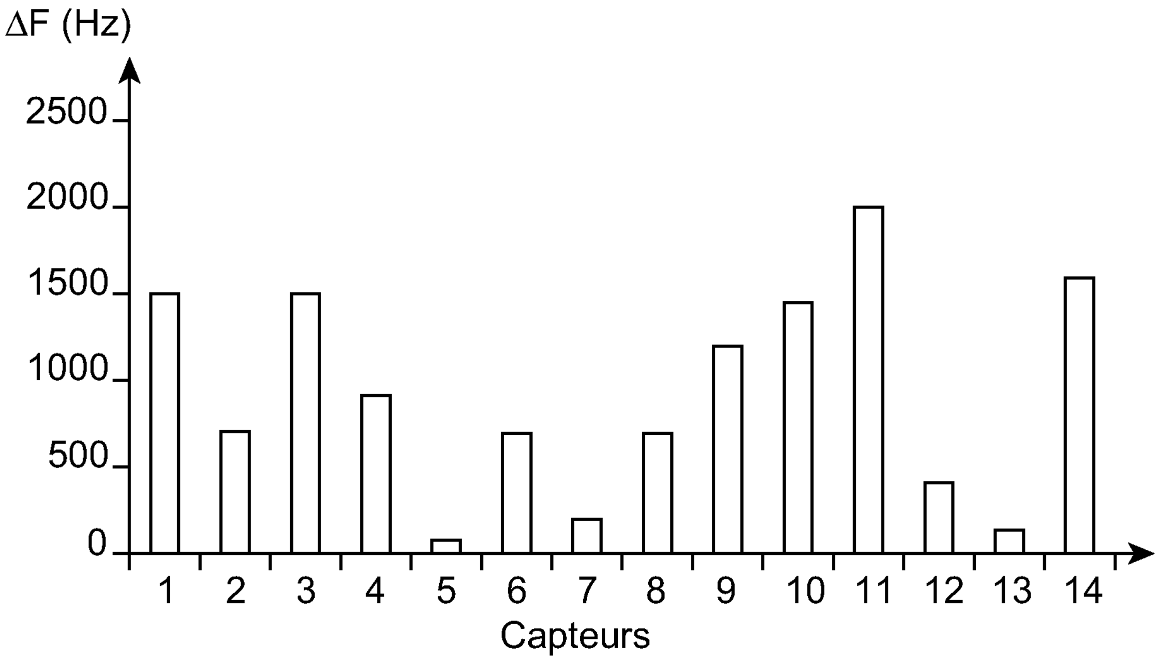
ΔF (Hz)
2500
2000
1500
1000
500
0
1
2
3
4
5
6
7
8
9
10
11
12
13
14
Capteurs

FIG. 1

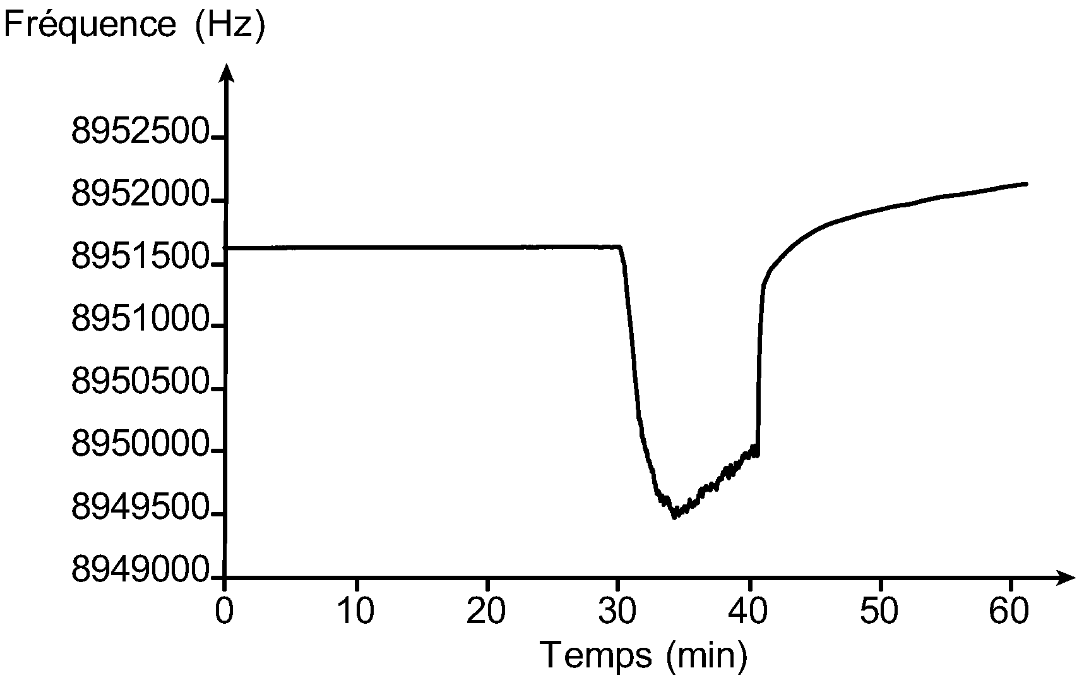
Fréquence (Hz)
8952500
8952000
8951500
8951000
8950500
8950000
8949500
8949000
0
10
20
30
40
50
60
Temps (min)

FIG. 2

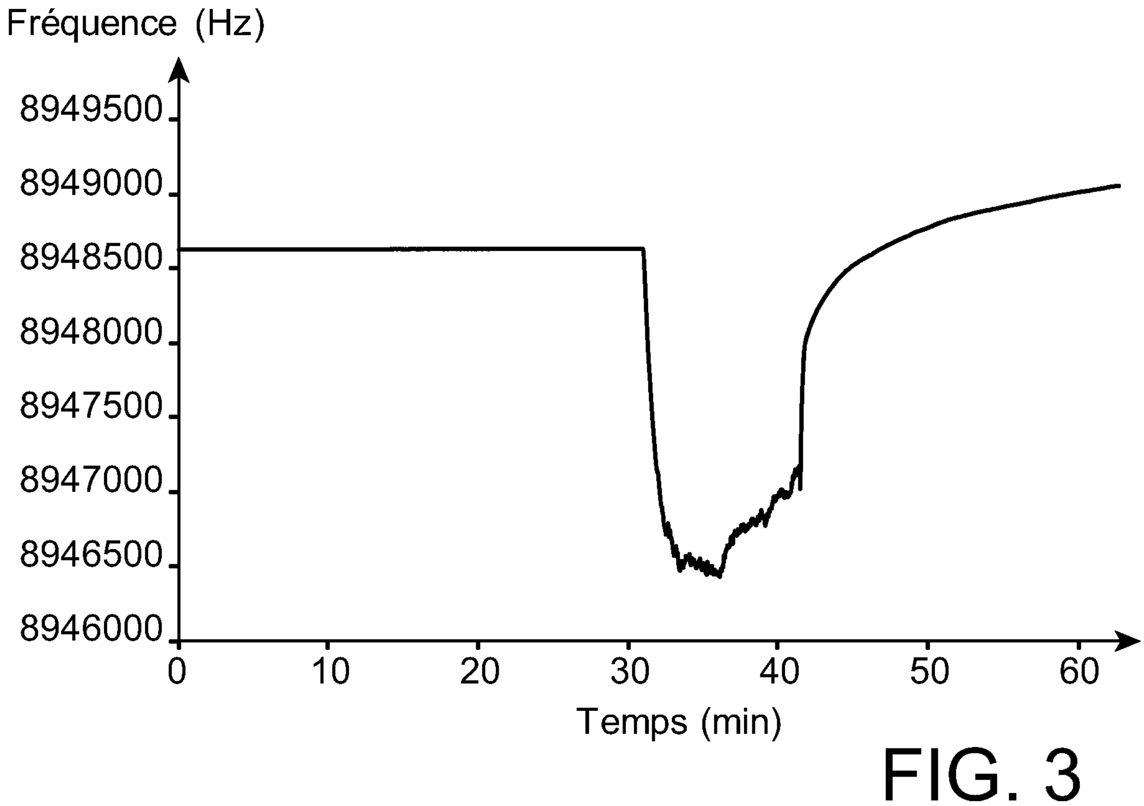
Fréquence (Hz)
8949500
8949000
8948500
8948000
8947500
8947000
8946500
8946000
0
10
20
30
40
50
60
Temps (min)

FIG. 3

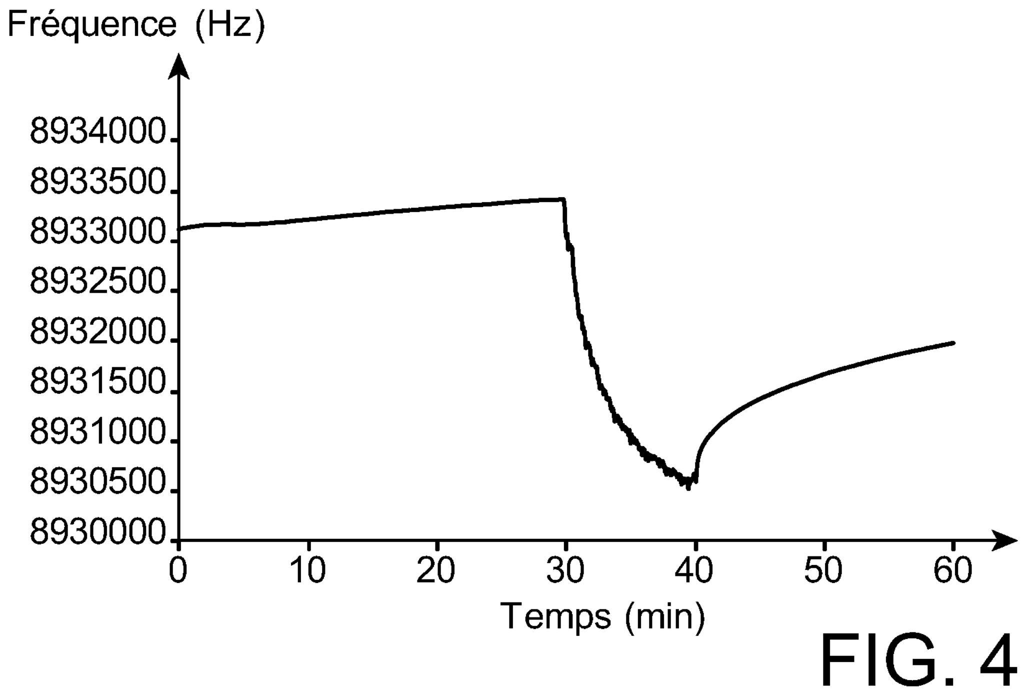
Fréquence (Hz)
8934000
8933500
8933000
8932500
8932000
8931500
8931000
8930500
8930000
0
10
20
30
40
50
60
Temps (min)

FIG. 4

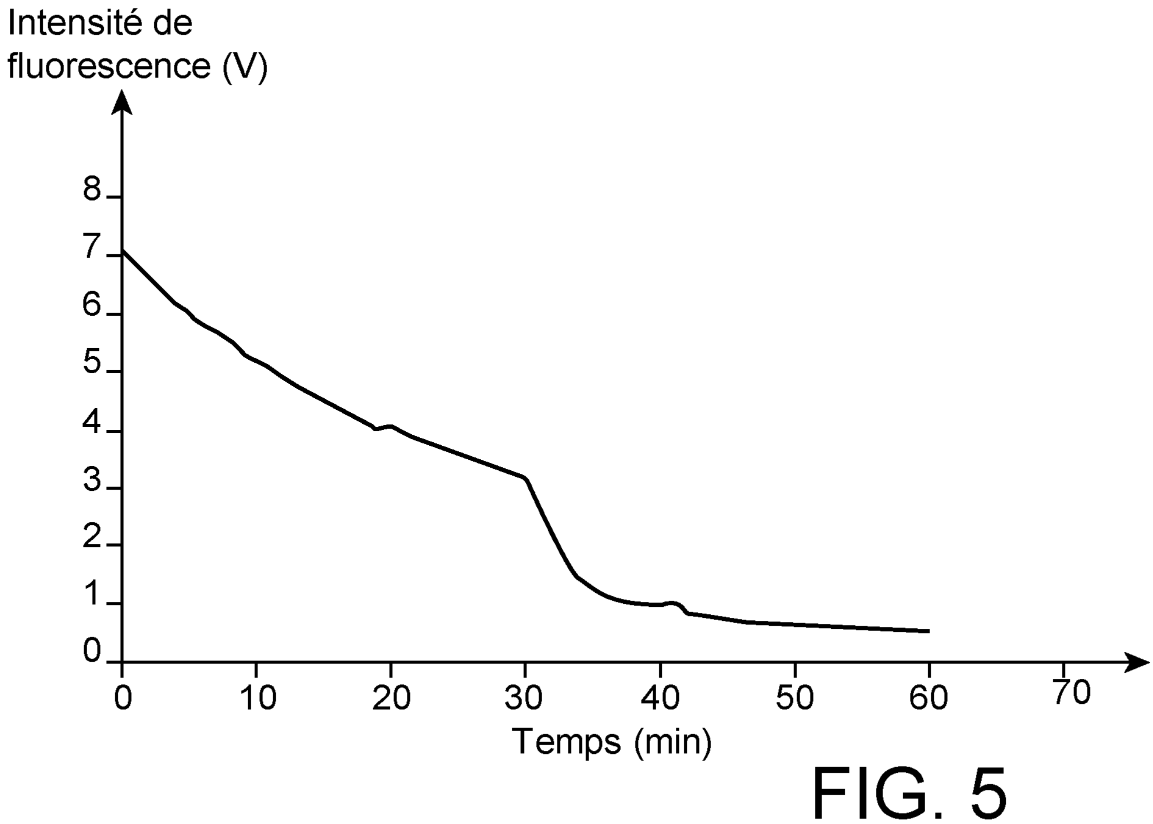
Intensité de
fluorescence (V)
8
7
6
5
4
3
2
1
0
0
10
20
30
40
50
60
70
Temps (min)

FIG. 5

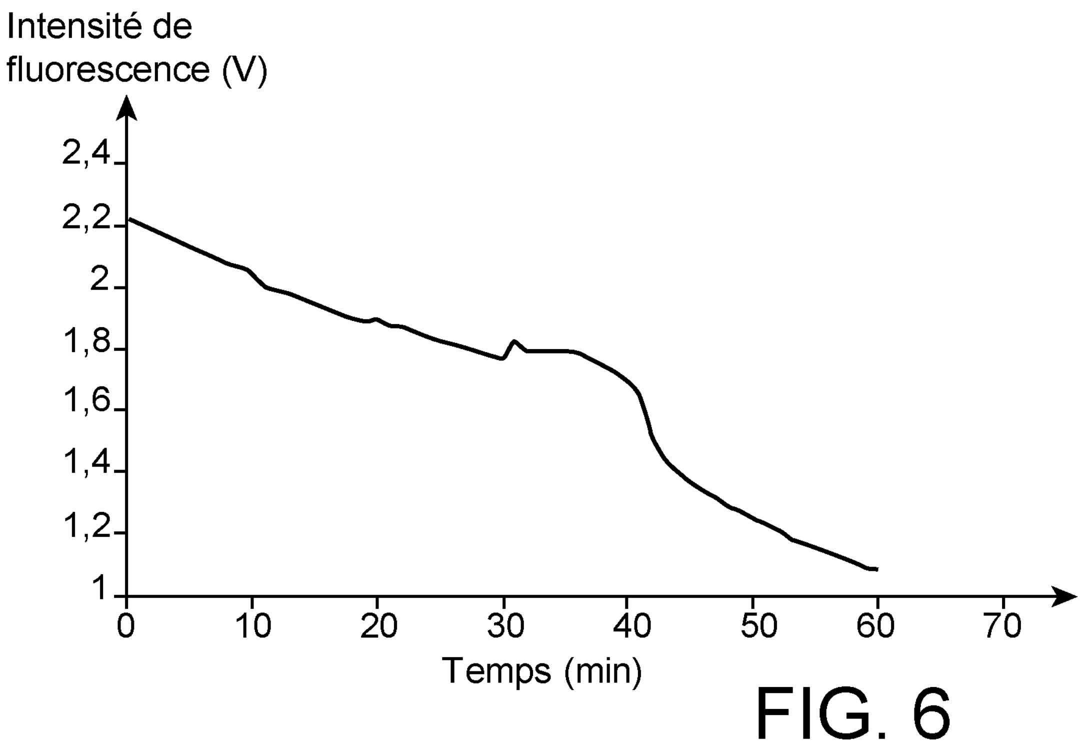
Intensité de
fluorescence (V)
2,4
2,2
2
1,8
1,6
1,4
1,2
1
0
10
20
30
40
50
60
70
Temps (min)

FIG. 6

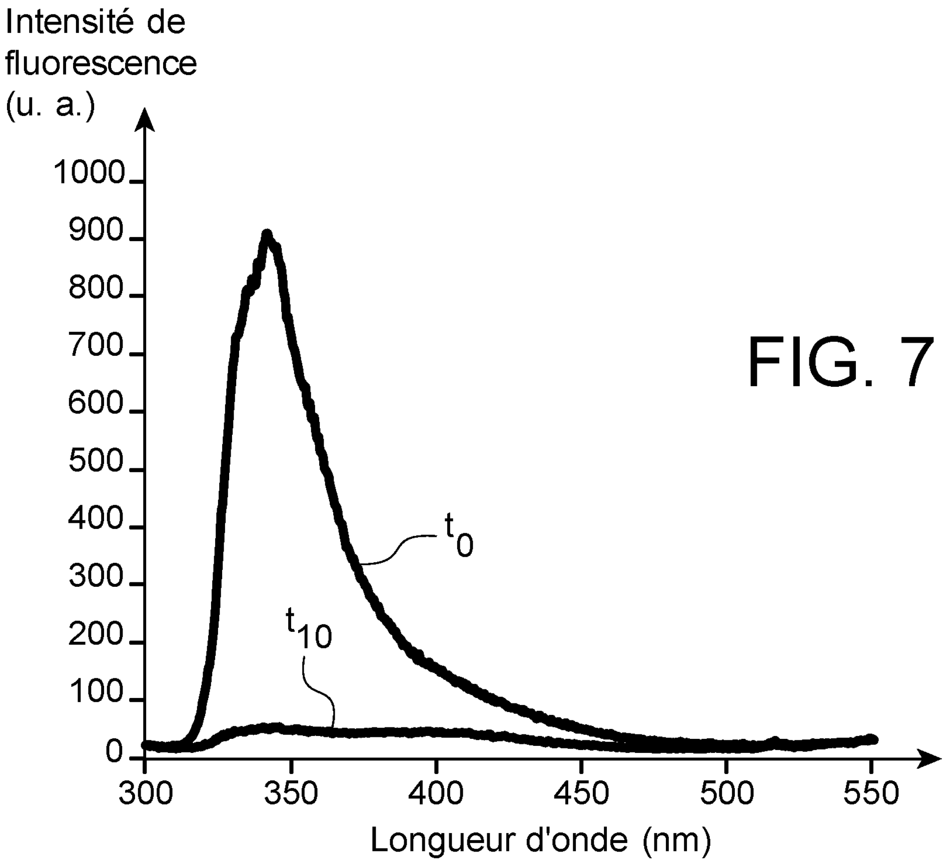
Intensité de fluorescence (u. a.)
1000
900
800
700
600
500
400
300
200
100
0
$t_0$
$t_{10}$
300
350
400
450
500
550
Longueur d'onde (nm)

FIG. 7

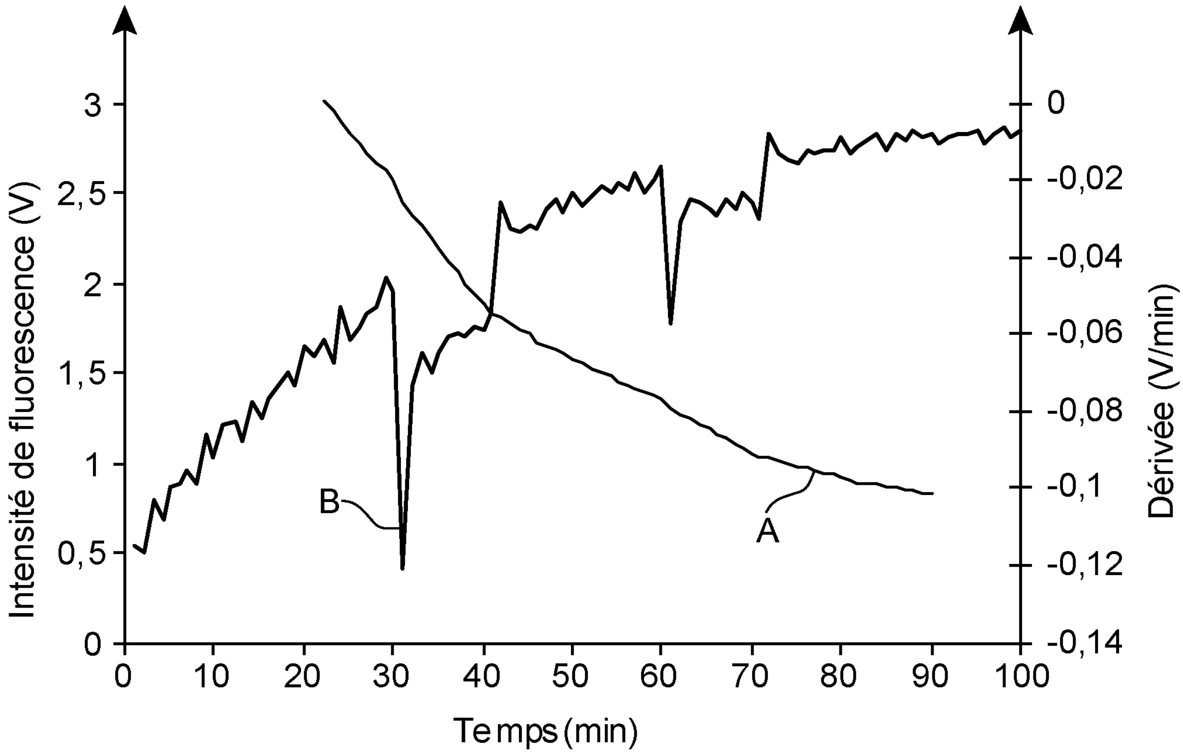
Intensité de fluorescence (V)
3
2,5
2
1,5
1
0,5
0
0
-0,02
-0,04
-0,06
-0,08
-0,1
-0,12
-0,14
Dérivée (V/min)
B
A
0
10
20
30
40
50
60
70
80
90
100
Temps (min)

FIG. 8

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

• WO 8304255 A

### Littérature non-brevet citée dans la description

• **J.A.O. SANCHEZ-PEDRONO et al.** *Anal. Chem. Acta,* 1986, vol. 182, 285